# EUROPEAN PATENT APPLICATION

(11) **EP 1 473 584 A1**
(43) Date of publication of application: **03.11.2004**
(21) Application number: 03737454.3
(22) Date of filing: 31.01.2003
(51) Int. Cl.: G02C 13/00, A61L 12/14

(54) **CONTACT LENS SOLUTION**

(30) Priority: 07.02.2002 JP 2002030285
(71) Applicant: Ophtecs Corporation, Osaka-shi, Osaka 550-0002 (JP)
(72) Inventor: FUJITA, Masaaki, c/o Ophtecs Corporation, Toyooka-shi, Hyogo 668-0831 (JP); NAKAMICHI, Chihiro, c/o Ophtecs Corporation, Toyooka-shi, Hyogo 668-0831 (JP); NISHIMURA, Shinji, c/o Ophtecs Corporation, Toyooka-shi, Hyogo 668-0831 (JP); UMEDA, Yuzuru, c/o Ophtecs Corporation, Toyooka-shi, Hyogo 668-0831 (JP); TAJIRI, Motoharu, c/o Ophtecs Corporation, Toyooka-shi, Hyogo 668-0831 (JP); SAITO, Fumio, c/o Ophtecs Corporation, Osaka-shi, Osaka 550-0002 (JP)
(74) Representative: Hallybone, Huw George
(86) International application number: PCT/JP2003/001001
(87) International publication number: WO 2003/067311

(57) **Abstract**

A contact lens solution containing polylysine, polyphosphoric acid and/or its salt preventing polylysine from adsorption by contact lenses, a nitrogen-containing organic antibacterial agent other than polylysine and water. Contact lenses can be washed, disinfected and preserved merely by soaking in the above solution without rubbing or rinsing before putting into eyes.

## Description

### Field of the Invention

The present invention relates to a liquid formulation for contact lenses. More specifically, it relates to a liquid formulation for contact lenses that is effective in cleaning, disinfecting and storing contact lenses.

### Prior Art

Since deposits such as protein derived from tears or lipid derived from discharge and so on generally adheres to the surfaces of contact lenses when the contact lenses are worn in eyes, the deposit must be removed after they are taken off from the eyes. When the contact lenses are kept used, while they are taken off from the eyes or stored in a lens case, the contamination of the contact lenses by microorganisms adhered to the contact lenses may occur. When the contaminated contact lenses are worn in eyes, a trouble may occur in the eyes. Therefore, it is important to disinfect the contact lenses before they are worn in eyes. It is said that the disinfection of soft contact lenses in particular is indispensable.

Methods of disinfecting contact lenses, particularly soft contact lenses are roughly divided into boiling disinfection methods and chemical disinfection methods. Recently, out of these methods, the chemical disinfecting methods are attracting much attention and becoming the main method of disinfecting contact lenses.

The chemical disinfection methods which are now becoming the main method of disinfecting soft contact lenses are further roughly divided into a method using a 3 % hydrogen peroxide solution, a method using so-called MPS which comprises a biguanide-based compound and polidronium chloride as disinfecting components, and a method using povidone-iodine. The method using MPS which is the mainstream in the chemical disinfection methods involves problems that it is unsatisfactory in terms of the disinfection of Candida and so on, that a trouble may occur in eyes when disinfected contact lenses are worn in eyes as a disinfecting component adheres to the contact lenses and that contact lenses must be cleaned by digital rubbing. Therefore, a disinfectant that has high safety and a sufficiently disinfecting effect and is easy to clean contact lenses, particularly a disinfecting product of MPS type has been sought for.

Recently, Polylysine, which is used as an agent for preserving food and so on, is attracting attention as a new disinfectant for contact lenses. Polylysine is a polymer of L-lysine that is an amino acid and has low toxicity to eyes and high safety as a disinfecting component of a liquid formulation for contact lenses. JP-A 8-504346 (the term "JP-A" as used herein means an "unexamined published Japanese patent application") discloses that polylysine greatly suppresses the formation of a protein deposit on a hydrophilic contact lens and/or within the lens. WO97/27879 discloses that polylysine is used as a solution for storing and disinfecting contact lenses. JP-A 2000-84052 discloses a liquid formulation for contact lenses that contains polylysine, amino acid and/or nonionic isotonic agent.

Out of these, JP-A 8-504346 mentions that polylysine has the property of adhering to contact lenses, particularly high water content ionic lenses which belong to group 4 in water content soft contact lenses (to be referred to as "group 4 lens" in the present invention). However, the above publication does not mention that polylysine adheres to group 4 lenses in large quantities and when the group 4 lenses to which polylysine adheres in large quantities are worn in eyes, they may cause a trouble in the eyes. This problem is extremely serious when the contact lenses are actually worn in eyes. Therefore, the above prior arts have poor practical applicability. Accordingly, the development of a liquid formulation for contact lenses that comprises polylysine as a disinfecting component, suppresses the adhesion of a large amount of polylysine to a contact lens and is safer for eyes has been desired.

### Summary of the Invention

It is therefore an object of the present invention to provide a liquid formulation for contact lenses which comprises polylysine as a disinfecting component, suppresses the adhesion of a large amount of polylysine to a contact lens and is safe for eyes.

Other objects and advantages of the present invention will become apparent from the following description.

According to the present invention, the above objects and advantages of the present invention are attained by a liquid formulation for contact lenses that comprises polylysine, polyphosphoric acid and/or salt thereof, nitrogen-containing organic anti-microbial agent (excluding polylysine) and water.

### Preferred Embodiment of the Invention

The liquid formulation for contact lenses of the present invention comprises polylysine as one of its disinfecting components. The polylysine may be either α-polylysine in which an amino group and a carboxyl group at the α-position are condensed, or ε-polylysine in which an amino group at the ε-position and a carboxyl group at the α-position are condensed. ε-Polylysine is particularly preferred. ε-Polylysine is available from Chisso Corporation, for example. The polylysine is contained in the formulation in an amount of preferably 1 x 10⁻⁴ to 2 (w/v) %, more preferably 1 x 10⁻³ to 0.2 (w/v) %, much more preferably 5 x 10⁻³ to 5 x 10⁻² (w/v) %.

The inventors of the present invention have made every effort to research and have found that polylysine has the property of adhering in large quantities to contact lenses, particularly a group 4 lens out of water content soft contact lenses and that when group 4 lenses to which polylysine adheres in large quantities are worn in eyes, they may cause a trouble in the eyes.

Therefore, the liquid formulation for contact lenses of the present invention contains a substance for suppressing the adhesion of polylysine to contact lenses as an essential ingredient based on the above discovered fact. As the substance for suppressing adhesion, it may be used polyphosphoric acid and/or salt thereof.

Preferred examples of the polyphosphoric acid include pyrophosphoric acid, tripolyphosphoric acid, trimetaphosphoric acid, tetraphosphoric acid and hexametaphosphoric acid. Preferred examples of the polyphosphate are salts of the above acids.

These polyphosphoric acids and salts thereof may be used alone or in combination of two or more.

The substance for suppressing adhesion is contained in the formulation in an amount of preferably 1 x 10⁻³ to 5 (w/v) %, more preferably 1 x 10⁻² to 2 (w/v) %, much more preferably 0.1 to 1 (w/v) %.

In the present invention, generally known nitrogen-containing organic anti-microbial agent is contained as a disinfectant for contact lenses besides polylysine. Preferred examples of the nitrogen-containing organic anti-microbial agent include polyhexamethylene biguanide, polyhexamethylene biguanide salt and quaternary ammonium salt. In the present invention, these nitrogen-containing organic anti-microbial agents may be used alone or in combination of two or more. Out of these, polyhexamethylene biguanide and/or salt thereof are particularly preferred. This disinfectant is contained in the formulation in an amount of preferably 1 x 10⁻⁶ to 1 (w/v) %, more preferably 1 x 10⁻⁵ to 1 x 10⁻² (w/v) %, much more preferably 5 x 10⁻⁵ to 5 x 10⁻⁴ (w/v) %.

The liquid formulation for contact lenses of the present invention may further contain water and at least one component selected from the group consisting of buffer, surfactant, viscosity inducing agent and isotonic agent. These components are described hereinbelow.

The buffer which can be contained in the liquid formulation for contact lenses of the present invention is not particularly limited if it is a buffer which is generally used as the formulation for contact lenses. When it is contained in the liquid formulation for contact lenses of the present invention, preferably, it does not reduce a disinfecting effect. Examples of the buffer include boric acid-based buffers and phosphoric acid-based buffers. As for other buffers, an effect obtained when each of the buffers is used in combination with polylysine and nitrogen-containing organic anti-microbial agent is confirmed in advance, and the combination of buffer is selected. If a disinfecting effect is not reduced when it is tested, there will be no problem in using another buffer. The buffer used in the present invention contains at least one from the above group. The buffer is contained in the formulation in an amount of preferably 1 x 10⁻² to 5 (w/v) %, more preferably 5 x 10⁻² to 2.5 (w/v) %, much more preferably 0.1 to 1.5 (w/v) %.

The surfactant that can be contained in the liquid formulation for contact lenses of the present invention is generally used as the formulation for contact lenses, which is, ampholytic surfactant, cationic surfactant, anionic surfactant and nonionic surfactant. Out of these, nonionic surfactant is particularly preferred. Examples of the nonionic surfactant include polyoxyethylene-polyoxypropylene block copolymer, polyoxyethylene alkyl ethers, polyoxyethylene alkylphenylethers, polyoxyethylene sorbitan alkylate, polyoxyethylene hydrogenated castor oil, monoglyceryl esters of fatty acids, propylene glycol esters of fatty acids, sucrose esters of fatty acids, polyoxyethylene-polyoxypropylene ethylenediamine condensates and so on. Examples of the cationic surfactant include tri(polyoxyethylene)stearylammonium chloride (5E.O.), oleylbis(2-hydroxyethyl)methylammonium chloride, N(N'-lanolin fatty acid amide propyl)N-ethyl-N,N-dimethylammonium ethyl sulfate, N-cocoyl-L-arginineethyl ester-DL-pyrrolidonecarboxylates and so on. Examples of the ampholytic surfactant include sodium lauryl diaminoethyl glycinate, 2-alkyl-N-carboxymethyl-N-hydroxyethyl imidazolinium betain, alkyldimethylamine oxide and so on. Examples of the anionic surfactant include α-olefin sulfonates, alkyl sulfonates, alkylbenzene sulfonates, polyoxyethylene carboxylated ether salts, alkyl phosphates, polyoxyethylene alkyl ether phosphates/sulfates, alkyl sulfates, alkyl ether sulfates, alkyl sarcosinates, alkyl methyl taurine, alkylbenzene sarcosinates and so on. In the present invention, the surfactant may be used alone or in combination of two or more. Preferably, an effect obtained when surfactant is used in combination with polylysine and/or nitrogen-containing organic anti-microbial agent is confirmed in advance, and the combination of surfactant is selected. When nonionic surfactant is used from among the above surfactant, it is contained in the formulation in an amount of 1 x 10⁻⁴ to 5 (w/v) %, preferably 1 x 10⁻³ to 2 (w/v) %, more preferably 1 x 10⁻² to 1 (w/v) %. When another surfactant is used, after an effect obtained when it is used in combination with polylysine and nitrogen-containing organic anti-microbial agent is confirmed in advance, it may be suitably used in limits that do not affect the effect.

The viscosity inducing agent that can be contained in the liquid formulation for contact lenses of the present invention is not particularly limited if it is generally used as the formulation for ophthalmology. Preferred examples of the viscosity inducing agent include hyaluronic acid and/or salt thereof, cellulose typified by hydroxypropylmethyl cellulose, and/or derivatives thereof, chitosan and/or derivatives thereof, polyvinylpyrrolidone, polyvinyl alcohols, chondroitin sulfuric acid and salt thereof and so on. In the present invention, the above viscosity inducing agents may be used alone or in combination of two or more. Preferably, an effect obtained when viscosity inducing agent is used in combination with polylysine and nitrogen-containing organic anti-microbial agent is confirmed in advance, and the combination of viscosity inducing agent is selected. The viscosity inducing agent is contained in the formulation in an amount of preferably 1 x 10⁻³ to 5 (w/v) %, more preferably 3 x 10⁻³ to 2.5 (w/v) %, much more preferably 5 x 10⁻³ to 1 (w/v) %.

The isotonic agent that can be contained in the liquid formulation for contact lenses of the present invention is not particularly limited if it is generally used as the formulation for contact lenses. Preferred examples of the isotonic agent include sodium chloride, potassium chloride, potassium iodide, glycerin, propylene glycol, polyethylene glycol, mannitol, sorbitol, dextrin, dextran and so on. In the present invention, the above isotonic agents may be used alone or in combination of two or more. Preferably, an effect obtained when isotonic agent is used in combination with polylysine and nitrogen-containing organic anti-microbial agent is confirmed in advance, and the combination of isotonic agent is selected. It is preferred that the isotonic agent may be suitably used so that the liquid formulation for contact lenses of the present invention can attain a targeted osmotic pressure.

The liquid formulation for contact lenses of the present invention may optionally contain metal chelating agent such as ethylenediamine tetraacetic acid (EDTA), citric acid, gluconic acid, tartaric acid, diethylenediamine pentaacetic acid, sodium or potassium salt thereof, and sodium nitrilotriacetate, a proteolytic enzyme derived from an animal, plant, microorganism and so on, and a pH adjustor such as hydrochloric acid and sodium hydroxide besides the above components. The liquid formulation for contact lenses of the present invention may contain at least one metal chelating agent, at least one proteolytic enzyme and at least one pH adjustor. Preferably, an effect obtained when they are used in combination with polylysine and nitrogen-containing organic anti-microbial agent is confirmed in advance, and the combination of them is selected.

The pH of the liquid formulation for contact lenses of the present invention is adjusted to a range of preferably 5.5 to 8.0, more preferably 6.8 to 7.8 by using the above pH adjustor and so on. The osmotic pressure of the liquid formulation is suitably selected from a range of preferably 180 to 460 mOsm., more preferably 260 to 360 mOsm.

The liquid formulation for contact lenses of the present invention is a liquid formulation obtained by dissolving the above components in a solvent that is harmless to eyes, such as purified water. It is particularly preferably a liquid formulation that contains the following components in purified water and has the above osmotic pressure and the above pH.
(A) 5 x 10⁻³ to 5 x 10⁻² (w/v) % of polylysine
(B) 0.1 to 1 (w/v) % of polyphosphate
(C) 5 x 10⁻⁵ to 5 x 10⁻⁴ (w/v) % of polyhexamethylene biguanide
(D) 0.1 to 1.5 (w/v) % of boric acid-based buffers
(E) 1 x 10⁻² to 1 (w/v) % of nonionic surfactant
(F) 5 x 10⁻³ to 1 (w/v) % of viscosity inducing agent

The liquid formulation for contact lenses of the present invention can be advantageously produced on an industrial scale, for example, by dissolving the above components excluding viscosity inducing agent in water, adjusting the pH of the obtained solution with acid or alkali, adding viscosity inducing agent, dissolving it under stirring for the night and finally adjusting the pH of the obtained solution with acid or alkali. To dissolve the above components, 5 to 15 (w/v) % aqueous solution of ε-polylysine and 10 to 30 (w/v) % solution of polyhexamethylene biguanide are prepared and mixed together in a predetermined ratio.

To clean, disinfect and store contact lenses using the liquid formulation for contact lenses of the present invention, the contact lenses taken off from eyes are soaked in the liquid formulation without cleaning by digital rubbing and left as they are for 5 minutes to 24 hours, preferably 30 minutes to 8 hours. Thereafter, the thus treated contact lenses can be worn in eyes without being rinsed with the liquid formulation. Although cleaning by digital rubbing which is generally necessary when MPS is used is not necessary in this method, when the contact lenses are very dirty, after they are taken off from eyes, they may be rinsed or cleaned by digital rubbing before they are soaked in the liquid formulation. When the liquid formulation is used as soaking liquid, after the contact lenses are cleaned with a commercially available general cleaning liquid or the like, they are soaked in the liquid formulation for 5 minutes or more and worn in eyes without being rinsed with the liquid formulation.

The contact lenses to be cleaned, disinfected and stored may be hard contact lenses and soft contact lenses of any one of groups 1 to 4. Soft contact lenses of group 4 are particularly preferred.

### Examples

The following examples and comparative examples are provided for the purpose of further illustrating the present invention but are not limited by these examples.

### Example 1

Solutions 1 to 3 and comparative solutions 1 to 3 having compositions shown in Table 1 below were prepared. The pH of these solutions and comparative solutions were adjusted by mixing an appropriate amount of hydrochloric acid or sodium hydroxide as a pH adjustor.

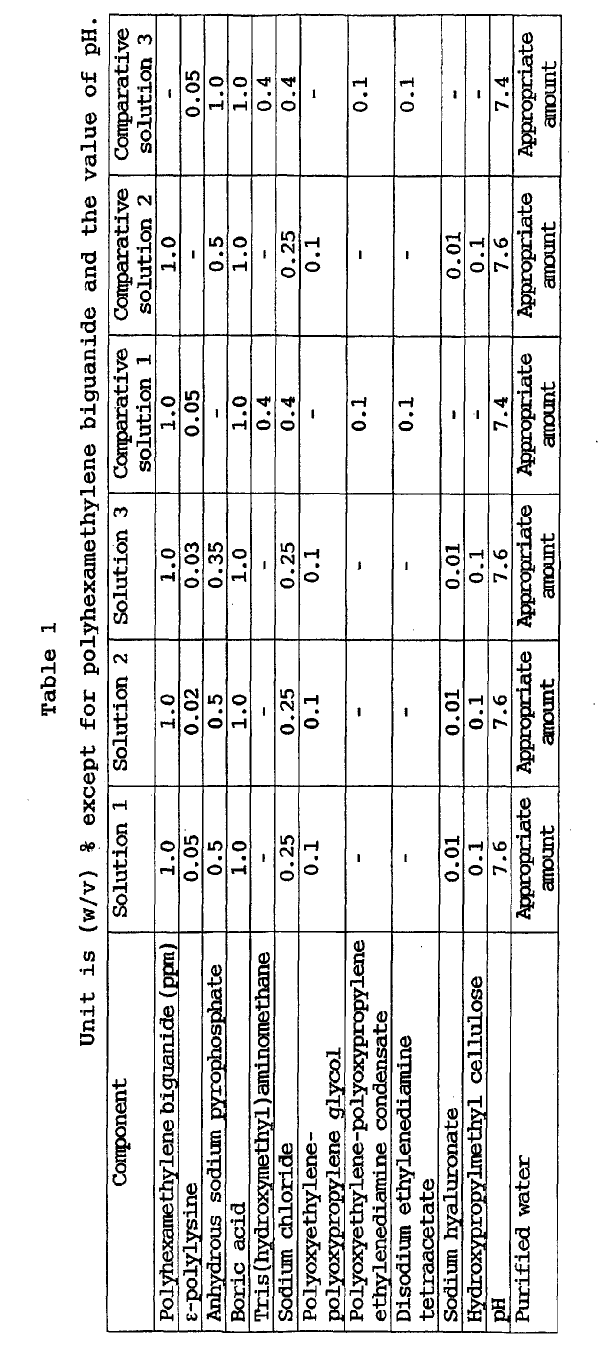

The amount of polylysine adhered to a contact lens was measured using the above solution 1, solution 2 and comparative solution 1 shown in Table 1. In this Example, "Surevue" (of Johnson and Johnson Co., Ltd.) which is a group 4 lens was used. For a single time of treatment, the lens was soaked in 4 ml of each solution and, at 25° C for 24 hours in solution 1 and solution 2 and for 8 hours in comparative solution 1. For 13 times of treatment, the lens was soaked in 4 ml of each solution, left at 25°C for 4 hours and then soaked in 1 ml of a phosphoric acid buffer physiologic saline specified in ISO10344 and shaken at 35° C for 2 hours or more. This operation was carried out 13 times for each solution. ε-Polylysine was extracted from the treated lens obtained by the above method and quantified by high performance liquid chromatography (of Shimadzu Corporation). The obtained results are shown in Table 2.

**Table 2**

| Unit: (µg/Lens) | | |
|---|---|---|
| | Number of times of treatment | Amount of adhered ε-polylysine |
| Solution 1 | One time | 14.04 ± 0.35 |
| | 13 times | 13.24 ± 1.41 |
| Solution 2 | One time | 6.23 ± 0.11 |
| | 13 times | 6.24 ± 0.11 |
| Comparative solution 1 | One time | 440.97 ± 13.14 |
| | 13 times | 459.84 ± 15.05 |

As shown in Table 2, the solution 1 and the solution 2 containing anhydrous sodium pyrophosphate which is a substance for suppressing the adhesion of ε-polylysine to a contact lens, greatly suppressed the adhesion of ε-polylysine to the contact lens, whereas a large amount of ε-polylysine adhered to a contact lens treated with the comparative solution 1 containing no anhydrous sodium pyrophosphate. The amount of adhered ε-polylysine was several ten times larger than those obtained by using the solution 1 and the solution 2. It was understood from the above results that the liquid formulation for contact lenses of the present invention has an excellent effect of suppressing the adhesion of ε-polylysine to a contact lens.

### Example 2

The influence upon human eyes of ε-polylysine when it adhered to contact lenses was investigated. In this Example, "Surevue" and "2 week Acuvue" (of Johnson and Johnson Co., Ltd.) both of which are group 4 contact lenses were used. These lenses were worn all day long, taken off from eyes, rinsed with the solution 1, solution 2, solution 3 and comparative solution 1 shown in Table 1 for 5 seconds or more and left in lens cases filled with the above solutions to be soaked in these solutions for the night, respectively. The following morning, the contact lenses were taken out from the lens cases and worn in the eyes without rinsing. The contact lenses were worn repeatedly for 2 weeks and treated with the solution 1, solution 2 or solution 3 every day. The contact lenses treated with the comparative solution 1 every day were to be worn in eyes for 2 weeks and if a problem occurred, their use was stopped. The results are shown in Table 3.

**Table 3**

| | Conditions |
|---|---|
| Solution 1 | No problem |
| Solution 2 | No problem |
| Solution 3 | No problem |
| Comparative solution 1 | Markedly conjunctival injection seen 1 hour after use |

As shown in Table 3, when the contact lenses treated with the comparative solution 1 were worn in healthy human eyes, the eyes became markedly conjunctival injection only in an hour. Further, it was judged that it was difficult for the eyes to continue wearing them and their use was stopped. It was understood from this result that as shown in Table 2, a large amount of ε-polylysine adheres to and accumulates on the contact lenses treated with the comparative solution 1 and may cause a trouble in eyes. In contrast to this, it was understood that when the contact lenses treated with the solution 1, solution 2 and solution 3 of the present invention are used for 2 weeks, no trouble occurs and the adhesion of ε-polylysine to the contact lenses is suppressed, thereby ensuring excellent safety.

### Example 3

A disinfecting effect test was carried out in accordance with an ISO stand-alone test using the solution 1, solution 2, solution 3, comparative solution 2 and comparative solution 3 shown in Table 1. The results are shown in Table 4.

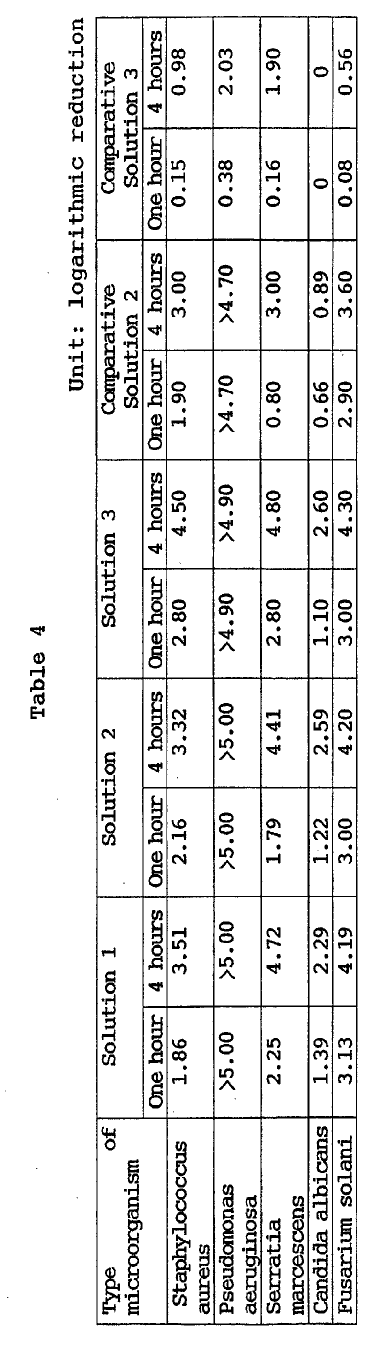

In Table 4, the larger the numerical value, the larger the disinfecting effect becomes.

As shown in Table 4, the solution 1, solution 2 and solution 3 showed a higher disinfecting effect than the comparative solution 2 and comparative solution 3 and came up to standards for all types of microorganism in 4 hours. It was understood from the above results that the liquid formulation for contact lenses of the present invention has an excellent disinfecting effect and practicability.

### Example 4

The cleaning effect of the liquid formulation for contact lenses of the present invention was confirmed from the cleaning effect of contact lenses that were artificially dirtied. In this example, the solution 3 shown in Table 1 and a commercially available product A comprising 0.7 ppm of polyhexamethylene biguanide as the comparative solution 4 were used and "2-week Acuvue" (of Johnson and Johnson Co., Ltd.) which is a group 4 lens was used as a contact lens.

The contact lenses were soaked in a 0.1 % lysozyme-containing physiological saline at 35°C for 16 hours to be dirtied artificially. Thereafter, the contact lenses were rinsed and soaked in the solution 3 at 25° C for 8 hours. As for the comparative solution 4, the contact lenses were cleaned by digital rubbing, rinsed in accordance with the directions and then soaked with the comparative solution 4 at 25° C for 8 hours. In addition, 13 cycles, each consisting of adhering dirt and treatment with each formulation, were made on the contact lenses. Six contact lenses were used to measure their initial absorbances (absorbance before treatment) and their absorbances after the 3rd treatment, 7th treatment and 13th treatment at a wavelength of 258.5 nm to obtain differences between the initial absorbance and absorbances after the 3rd, 7th and 13th treatments of the contact lenses. The average value of the differences was obtained. The results are shown in Table 5.

**Table 5**

| Number of times of treatment | Solution 3 | Comparative solution 4 |
|---|---|---|
| 3 times | 0.6116 ± 0.0424 | 0.7331 ± 0.0492 |
| 7 times | 0.6845 ± 0.0610 | 0.9110 ± 0.0685 |
| 13 times | 0.6849 ± 0.0592 | 0.9905 ± 0.0824 |

As shown in Table 5, an excellent cleaning effect was obtained after 3 times, 7 times and 13 times of treatment were carried out. When a t-test was made on these results, there was a significant difference at p ≦ 0.001 in all the treatments, which means that the solution 3 had a significantly higher cleaning effect than the comparative solution 4. It was understood from this result that the liquid formulation for contact lenses of the present invention has an excellent cleaning effect without cleaning by digital rubbing.

### Industrial feasibility

As obvious from the above description, since the liquid formulation for contact lenses of the present invention comprises polylysine, polyphosphoric acid and/or salt thereof as a substance for suppressing the adhesion of polylysine to a contact lens, nitrogen-containing organic anti-microbial agent excluding polylysine and water in a predetermined ratio, it can clean deposits adhered to the contact lens, disinfect it from microorganisms and store it. There can be further provided a liquid formulation for contact lenses having high safety for eyes by suppressing the adhesion of polylysine to the contact lens. Cleaning, disinfection and storing of contact lenses can be carried out simply by soaking the contact lenses in the liquid formulation without cleaning by digital rubbing that is usually necessary when MPS is used and without rinsing before the contact lenses are worn in eyes. Therefore, there can be provided a liquid formulation for contact lenses with which contact lenses can be treated very easily.

## Claims

1. A liquid formulation for contact lenses which comprises polylysine, polyphosphoric acid and/or salt thereof, nitrogen-containing organic anti-microbial agent (excluding polylysine) and water.

2. The liquid formulation for contact lenses according to claim 1, wherein the polylysine is ε-polylysine.

3. The liquid formulation for contact lenses according to claim 1, wherein the polyphosphoric acid is at least one selected from the group consisting of pyrophosphoric acid, tripolyphosphoric acid, trimetaphosphoric acid, tetraphosphoric acid, hexametaphosphoric acid and a combination thereof, and the polyphosphate is the salt of at least one of them.

4. The liquid formulation for contact lenses according to claim 1, wherein the nitrogen-containing organic anti-microbial agent is at least one selected from the group consisting of polyhexamethylene biguanide, polyhexamethylene biguanide salt and quaternary ammonium salt.

5. The liquid formulation for contact lenses according to claim 1, wherein the amount of polylysine is 1 x 10⁻⁴ to 2 (w/v) %, the amount of the polyphosphoric acid and/or salt thereof is 1 x 10⁻³ to 5 (w/v) % and the amount of the nitrogen-containing organic anti-microbial agent is 1 x 10⁻⁶ to 1 (w/v) % based on the liquid formulation.

6. The liquid formulation for contact lenses according to claim 1, which further comprises at least one additive, selected from the group consisting of buffer, surfactant, viscosity inducing agent and isotonic agent.

7. The liquid formulation for contact lenses according to claim 1, which is used for water content contact lenses.
